(19) **Europäisches Patentamt / European Patent Office / Office européen des brevets**

(11) **EP 1 135 128 B1**

(12) **EUROPEAN PATENT SPECIFICATION**

(45) Date of publication and mention of the grant of the patent:
**11.07.2007 Bulletin 2007/28**

(21) Application number: **99960293.1**

(22) Date of filing: **12.11.1999**

(51) Int Cl.:
*A61K 31/44* (2006.01)     *A61K 31/425* (2006.01)

(86) International application number:
**PCT/US1999/026746**

(87) International publication number:
**WO 2000/027341 (18.05.2000 Gazette 2000/20)**

(54) **NOVEL METHOD OF TREATMENT**

NEUE BEHANDLUNGSMETHODE

NOUVELLE METHODE THERAPEUTIQUE

(84) Designated Contracting States:
**AT BE CH CY DE DK ES FI FR GB GR IE IT LI LU MC NL PT SE**
Designated Extension States:
**AL LT LV MK RO SI**

(30) Priority: **12.11.1998 GB 9824893**

(43) Date of publication of application:
**26.09.2001 Bulletin 2001/39**

(60) Divisional application:
**06124048.7 / 1 759 698**

(73) Proprietor: **SMITHKLINE BEECHAM CORPORATION**
**Philadelphia, PA 19103 (US)**

(72) Inventors:
 • **BENINCOSA, Lisa**
 **Collegeville, PA 19426 (US)**
 • **JUSKO, William**
 **East Amherst, NY 14051 (US)**

(74) Representative: **Rutter, Keith**
**GlaxoSmithKline**
**Corporate Intellectual Property (CN9.25.1)**
**980 Great West Road**
**Brentford, Middlesex TW8 9GS (GB)**

(56) References cited:
| | |
|---|---|
| **EP-A- 0 008 203** | **EP-A- 0 032 128** |
| **EP-A- 0 155 845** | **EP-A- 0 177 353** |
| **EP-A- 0 428 312** | **EP-A- 0 489 663** |
| **EP-A1- 0 419 035** | **WO-A1-94/05659** |
| **US-A- 5 002 953** | **US-A- 5 457 109** |
| **US-A- 5 478 852** | **US-A- 5 478 853** |
| **US-A- 5 506 245** | **US-A- 5 708 012** |
| **US-A- 5 900 435** | **US-A- 5 902 726** |
| **US-A- 5 972 973** | **US-A- 6 011 049** |

Remarks:
The file contains technical information submitted after the application was filed and not included in this specification

**Description**

**FIELD OF THE INVENTION**

This invention relates to a pharmaceutical composition useful in a method for the treatment of Type 2 diabetes mellitus and conditions associated with diabetes mellitus.

**BACKGROUND OF THE INVENTION**

European Patent Application, Publication Number 0,306,228 relates to certain thiazolidinedione derivatives disclosed as having antihyperglycaemic and hypolipidaemic activity. One particular thiazolidinedione disclosed in EP 0306228 is 5-[4-[2-(N-methyl-N-(2-pyridyl)amino)ethoxy]benzyl]thiazolidine-2,4-dione (hereinafter "Compound (I)"). WO94/05659 discloses certain salts of Compound (I) including the maleate salt at example 1 thereof.

Compound (I) is an example of a class of anti-hyperglycaemic agents known as "insulin sensitisers". In particular Compound (I) is a thiazolidinedione insulin sensitiser.

European Patent Applications, Publication Numbers: 0008203, 0139421, 0032128, 0428312, 0489663, 0155845, 0257781, 0208420, 0177353, 0319189, 0332331, 0332332, 0528734, 0508740; International Patent Application, Publication Numbers 92/18501, 93/02079, 93/22445 and United States Patent Numbers 5104888 and 5478852, also disclose certain thiazolidinedione insulin sensitisers.

It is now surprisingly indicated that the particular plasma concentrations of Compound (I), which provide effective glycaemic control, indeed an optimum effect on glycaemic control, can be determined. This therefore enables optimization of the dosing regimen for the anti-diabetic agent for a given dosing interval. Pharmaceutical compositions which provide plasma concentrations of

Compound (I), at these particular concentrations, especially over an extended period of time, are envisaged by this invention.

**SUMMARY OF THE INVENTION**

Accordingly, in a first aspect, the present invention provides a sustained release pharmaceutical composition in unit dosage form and adapted for oral administration comprising 2 to 12 mg of 5-[4-[2-(N-methyl-N-(2-pyridyl)amino) ethoxy]benzyl]thiazolidine-2,4-dione or a pharmaceutically acceptable salt or solvate thereof and a pharmaceutically acceptable carrier therefor, which dosage form provides a plasma concentration of 5-[4-[2-(N-methyl-N-(2-pyridyl)amino) ethoxy]benzyl]thiazolidine-2,4-dione of a Threshold Plasma Concentration, which is at least 50 ng/mL over a period of up to 24 hours.

**DETAILED DESCRIPTION OF THE INVENTOIN**

The Threshold Plasma Concentration is suitably within the range of from 50 to 200ng/nL, including 50 to 120ng/mL, 60 to 120ng/mL, 90 to 110ng/mL or 95 to 105ng/mL.

A suitable minimum Threshold Plasma Concentration (hereinafter "Minimum Threshold Plasma Concentration") is the SC50 concentration of Compound (I) is 50 or, more suitably, 51.4ng/mL.

A preferred Threshold Plasma Concentration (hereinafter "Preferred Threshold Plasma Concentration") is twice the SC50 concentration, which for Compound (I) is in the range of 80 to 130 ng/mL, more suitably 82.2 to 123.4, for example 100 ng/mL or 102.8 ng/mL.

The invention particularly envisages compositions useful in treatments wherein the plasma concentration of the insulin sensitiser remains substantially within the range of concentrations from the Minimum Threshold Plasma Concentration to the Preferred Threshold Plasma Concentration, that is for Compound (I) within the range of from 50ng/mL to 100ng/mL or 51.4ng/mL to 102.8 ng/mL.

The invention also particularly envisages compositions useful in treatments wherein the plasma concentration of the insulin sensitiser remains substantially within the range of concentrations from the Minimum Threshold Plasma Concentration to a level at or above the Preferred Threshold Plasma Concentration, that is for Compound (I) within the range of from 50ng/mL to 100ng/mL or 51.4 ng/mL to a level at or above 102.8 ng/mL.

In its preferred form, the invention provides compositions useful in a treatment wherein the plasma concentration of the insulin sensitiser remains substantially at or above the Preferred Threshold Plasma Concentration, that is for Compound (I), substantially at or above 100ng/mL, especially substantially at or above 102.8ng/mL.

The unit dose suitably comprises 2 to 12 or preferably 4 to 8 mg of Compound (I) in a pharmaceutically acceptable form.

As indicated above, the invention is a pharmaceutical composition of compound (I) or a pharmaceutically acceptable salt or solvate thereof adapted so as to provide a plasma concentration of at least a Threshold Plasma Concentration of compound (I).

The carrier is adapted to provide the provide a plasma concentration of compound (I) of at least a Threshold Plasma Concentration.

The invention particularly envisages compositions adapted to provide a plasma concentration of the insulin sensitiser which remains substantially within the range of concentrations from the Minimum Threshold Plasma Concentration to the Preferred Threshold Plasma Concentration, that is for Compound (I) within the range of from 50 to 100ng/mL or 51.4 to 102.8 ng/mL.

The invention also envisages compositions adapted to provide a plasma concentration of the insulin sensitiser which remains substantially at or above the Preferred Threshold Plasma Concentration, that is for Compound (I), substantially at or above 100ng/mL, especially substantially at or above 102.8ng/mL.

The composition is a unit dose composition.

Suitably, the Threshold Plasma concentration of Compound (I) is maintained or exceeded over several hours, for example 12, 16 or 24 hours, per dose of insulin sensitiser.

Suitably, the treatment is such that the Threshold Plasma concentration of Compound (I) is maintained or exceeded over a sustained period of time.

It will be understood that Compound (I) is administered in a pharmaceutically acceptable form, including pharmaceutically acceptable derivatives such as pharmaceutically acceptable salts, esters and solvates thereof, as appropriate of the relevant pharmaceutically active agent. It will be understood that all pharmaceutically acceptable forms of the active agents per se are encompassed by this invention.

Suitable pharmaceutically acceptable salted forms of Compound (I) include those described in EP 0306228 and WO94/05659. A preferred pharmaceutically acceptable salt is a maleate.

Suitable pharmaceutically acceptable solvated forms of Compound (I) include those described in EP 0306228 and WO94/05659, in particular hydrates.

Compound (I) or, a pharmaceutically acceptable salt thereof, or a pharmaceutically acceptable solvate thereof, may be prepared using known methods, for example those disclosed in EP 0306228 and WO94/05659. The disclosures of EP 0306228 and WO94/05659 are incorporated herein by reference.

Compound (I) may exist in one of several tautomeric forms, all of which are encompassed by the term Compound (I) as individual tautomeric forms or as mixtures thereof. Compound (I) contains a chiral carbon atom, and hence can exist in up to two stereoisomeric forms, the term Compound (I) encompasses all of these isomeric forms whether as individual isomers or as mixtures of isomers, including racemates.

When used herein the term "conditions associated with diabetes" includes those conditions associated with the pre-diabetic state, conditions associated with diabetes mellitus itself and complications associated with diabetes mellitus.

When used herein the term "conditions associated with the pre-diabetic state" includes conditions such as insulin resistance, including hereditary insulin resistance, impaired glucose tolerance and hyperinsulinaemia.

"Conditions associated with diabetes mellitus itself' include hyperglycaemia, insulin resistance, including acquired insulin resistance and obesity. Further conditions associated with diabetes mellitus itself include hypertension and cardiovascular disease, especially atherosclerosis and conditions associated with insulin resistance. Conditions associated with insulin resistance include polycystic ovarian syndrome and steroid induced insulin resistance and gestational diabetes.

"Complications associated with diabetes mellitus" includes renal disease, especially renal disease associated with Type II diabetes, neuropathy and retinopathy.

Renal diseases associated with Type II diabetes include nephropathy, glomerulonephritis, glomerular sclerosis, nephrotic syndrome, hypertensive nephrosclerosis and end stage renal disease.

As used herein the term 'pharmaceutically acceptable' embraces both human and veterinary use: for example the term pharmaceutically acceptable" embraces a veterinarily acceptable compound.

When used herein the term "SC50 concentration" refers to the plasma concentration for a given compound required to produce a half-maximal effect on fasting plasma glucose for that compound.

For the avoidance of doubt, when reference is made herein to scalar amounts, including mg amounts, of Compound (I) in a pharmaceutically acceptable form, the scalar amount referred to is made in respect of Compound (I) *per se:* For example 2 mg of Compound (I) in the form of the maleate salt is that amount of maleate salt which contains 2 mg of Compound (I).

Diabetes mellitus is preferably Type II diabetes.

Glycaemic control may be characterised using conventional methods, for example by measurement of a typically used index of glycaemic control such as fasting plasma glucose or glycosylated haemoglobin (Hb A1c). Such indices are determined using standard methodology, for example those described in: Tuescher A, Richterich, P., Schweiz. med. Wschr. 101 (1971), 345 and 390 and Frank P., "Monitoring the Diabetic Patent with Glycosolated Hemoglobin Measurements", Clinical Products 1988.

Preferably, the treatment using the invention will effect an improvement in the levels of advanced glycosylation end products (AGEs), leptin and serum lipids including total cholesterol, HDL-cholesterol, LDL-cholesterol including improvements in the ratios thereof, in particular an improvement in serum lipids including total cholesterol, HDL-cholesterol, LDL-cholesterol including improvements in the ratios thereof.

Usually the compositions are adapted for oral administration. However, they may be adapted for other modes of administration, for example parenteral administration, sublingual or transdermal administration.

The compositions may be in the form of tablets, capsules, powders, granules, lozenges, suppositories, reconstitutable powders or liquid preparations, such as oral or sterile parenteral solutions or suspensions.

In order to obtain consistency of administration a composition of the invention is in the form of a unit dose.

Unit dosage presentation forms for oral administration may be in tablet or capsule form and may as necessary contain conventional excipients such as binding agents, fillers, lubricants, glidants, disintegrants and wetting agents.

Examples of binding agents include acacia, alginic acid, carboxymethylcellulose calcium, carboxymethylcellulose sodium, dextrates, dextrin, dextrose, ethylcellulose, gelatin, liquid glucose, guar gum, hydroxyethyl cellulose, hydroxypropyl cellulose, hydroxypropyl methylcellulose, magnesium aluminium silicate, maltodextrin, methyl cellulose, polymethacrylates, polyvinylpyrrolidone, pregelatinised starch, sodium alginate, sorbitol, starch, syrup, tragacanth.

Examples of fillers include calcium carbonate, calcium phosphate, calcium sulphate, carboxymethylcellulose calcium, carboxymethylcellulose sodium, compressible sugar, confectioner's sugar, dextrates, dextrin, dextrose, dibasic calcium phosphate dihydrate, dibasic calcium phosphate, fructose, glyceryl palmitostearate, glycine, hydrogenated vegetable oil-type 1, kaolin, lactose, maize starch, magnesium carbonate, magnesium oxide, maltodextrin, mannitol, microcrystalline cellulose, polymethacrylates, potassium chloride, powdered cellulose, pregelatinised starch, sodium chloride, sorbitol, starch, sucrose, sugar spheres, talc, tribasic calcium phosphate, xylitol.

Examples of lubricants include calcium stearate, glyceryl monostearate, glyceryl palmitostearate, magnesium stearate, microcrystalline cellulose, sodium benzoate, sodium chloride, sodium lauryl sulphate, stearic acid, sodium stearyl fumarate, talc, zinc stearate.

Examples of glidants include colloidal silicon dioxide, powdered cellulose, magnesium trisilicate, silicon dioxide, talc.

Examples of disintegrants include alginic acid, carboxymethylcellulose calcium, carboxymethylcellulose sodium, colloidal silicon dioxide, croscarmellose sodium, crospovidone, guar gum, magnesium aluminium silicate, microcrystalline cellulose, methyl cellulose, polyvinylpyrrolidone, polacrilin potassium, pregelatinised starch, sodium alginate, sodium lauryl sulphate, sodium starch glycollate.

An example of a pharmaceutically acceptable wetting agent is sodium lauryl sulphate.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agent can be dissolved in the vehicle. To enhance the stability, the composition can be frozen after filling into the vial and the water removed under vacuum. Parenteral suspensions are prepared in substantially the same manner, except that the Compound (I) suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

As indicated the compositions are in a unit dosage form in an amount appropriate for the relevant daily dosage.

The present compositions may also contain other medicaments in addition to insulin sensitisers including other anti diabetic agents, such as insulin secretagogues, biguanide antihyperglycaemic agents and alpha glucosidase inhibitor antihyperglycaemic agents.

The medicaments may be administered from 1 to 6 times a day, suitably 1 or 2 times per day, preferably once per day.

Compositions may contain from 0.1% to 99% by weight, preferably from 10-60% by weight, of the active material, depending upon the method of administration.

The composition may, if desired, be in the form of a pack accompanied by written or printed instructions for use.

The method by which the Threshold Plasma Concentration, such as the SC50 concentration, for a given compound can be determined is:

1) first to obtain plasma concentrations versus time data for the compound, preferably using data from humans, by using standard pharmacokinetic compartmental modelling methods (for example for Compound (I), concentrations were fit to a one compartment model.);

2) the model predicted concentrations for the compound are then fed back into the model and used to determine the change in fasting plasma glucose levels after various doses;

3) the relationship between predicted plasma concentrations of compound and fasting plasma glucose can suitably be determined using an indirect pharmacological response model (model IV), for example that described in (Dayneka NL, Garg V and Jusko WJ, Comparison of Four Basic Models of Indirect Pharmacodynamic Responses. J of Pharmacokinetics and Biopharmaceutics. Vol 21, No 4. 1993). This model yields estimates of glucose input rate (Kin) and output rate (Kout), maximal stimulation of glucose output (Smax). Hill coefficient (gamma) and the Threshold Plasma Concentration, such as the SC50 concentration (i.e the concentration associated with a half maximal response) to be determined for that compound.

For Compound (I), it was necessary to account for a time delay between the time of actual initiation of dosing (week 0) and the observed change in fasting plasma glucose. This delay factor was estimated through the modelling for each dose level. The mean delay across dose levels for Compound (I) was found to be 292 hours. The delay factor was incorporated into the model for deriving fasting plasma glucose by assuming that the first dose of drug occurred only after the time dictated by the delay factor. It is considered that for this model a delay factor would be required for other thiazolidinedione insulin sensitisers and that this factor will be substantially similar to that found for Compound (I). Delay factors for other compounds may also be required to be determined using similar methodology to that disclosed herein.

Compositions of the invention can be prepared by a process for preparing a pharmaceutical composition comprising compound (I) and a pharmaceutically acceptable carrier therefor, comprising formulating compound (I) and the pharmaceutically acceptable carrier so as to provide a plasma concentration of the insulin sensitiser of at least a Threshold Plasma Concentration of compound (I).

The carrier is adapted so as to provide a plasma concentration of compound (I) of at least a Threshold Plasma Concentration.

The compositions are prepared and formulated according to conventional methods, such as those disclosed in standard reference texts, for example the British and US Pharmacopoeias, Remington's Pharmaceutical Sciences (Mack Publishing Co.), Martindale The Extra Pharmacopoeia (London, The Pharmaceutical Press) (for example see the 31 st Edition page 341 and pages cited therein) and Harry's Cosmeticology (Leonard Hill Books) or the above mentioned publications.

The modified release compositions may be formulated according to appropriate methods disclosed in for example Sustained and Controlled Release Drug Delivery Systems, Editor Joe R Robinson, Volume 7, published by Marcel Dekker under the title Drugs and the Pharmaceutical Sciences, Controlled Drug Delivery, 2nd Edition' edited by Joe Robinson and Vince Lee, Marcel Dekker, 1987 and 'Drug Delivery to the Gastrointestinal Tract' Editors: J G Hardy, S S. Davis and C G Wilson also with reference to texts such as the British and US Pharmacopoeias, Remington's Pharmaceutical Sciences (Mack Publishing Co.), Martindale The Extra Pharmacopoeia (London, The Pharmaceutical Press) (for example see the 31st Edition page 341 and pages cited therein) and Harry's Cosmeticology (Leonard Hill Books).

No adverse toxicological effects are expected for the compositions of the invention in the above mentioned dosage ranges.

**EXAMPLES**

**Example: Pharmacokinetic/Pharmacodynamic Modeling of Compound (I) in Type 2 Diabetes Patients**

A PK/PD model was developed to characterise the effect of Compound (I) on fasting plasma glucose (FPG) concentrations in diabetic patients. The model was developed using mean fasting plasma glucose data from a Phase III clinical trial which consisted of comparison of placebo and four doses/regimens of Compound (I) in a parallel-group design of 26 weeks duration. Dose regimens evaluated were: 4 mg once daily and 2 mg twice daily, and 8 mg once daily and 4 mg twice daily.

Pharmacokinetics of Compound (I)

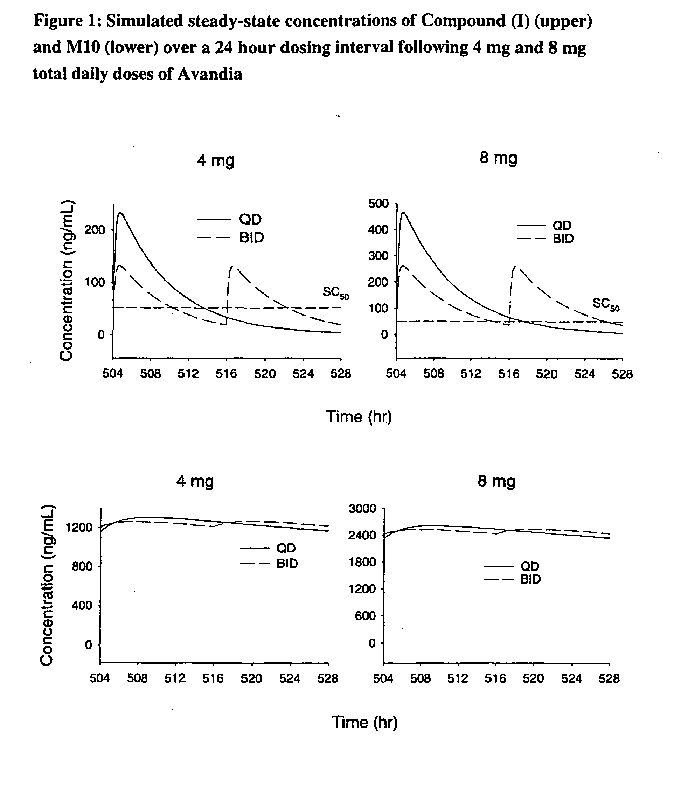

The pharmacokinetics of Compound (I) were described using a one-compartment model with first order oral absorption. Individual bayesian estimates of Compound (I) oral clearance and steady-state volume of distribution were predicted for each patient in the same Phase III clinical trial utilizing the population parameter estimates (priors) from the Phase I population pharmacokinetic analysis. Mean concentration-time profiles (Cp) for each regimen for use in the pharmacodynamic modeling were predicted using the mean post hoc oral clearance (2.68 L/h) and Vss/F (15.4 L) values across these patients (Figure 1).

Pharmacodynamics of Compound (I)

A modified indirect response model IV (Dayneka et al. 1993) was developed utilizing the pharmacokinetics of Compound (I) as the driving force for the change in fasting plasma glucose after various doses of Compound (I). Modeling fittings were done using ADAPT II, Release 4 (D'Argenio and Schumitzky,1979).

In the absence of Compound (I), plasma glucose levels are governed by formation (kin) and utilization (kout). The action of Compound (I) was described as stimulation (S(t)) of the utilization of plasma glucose (kout), described in text as FPG reduction (Eq. 1). $S_{max}$ represents the maximal stimulation, $SC_{50}$ is the Compound (I) concentration associated with half-maximal stimulation and $\gamma$ represents a sigmoidicity parameter in the Hill type function (Eq. 2).

$$\frac{dFPG}{dt} = k_{in} - k_{out} \bullet S(t) \bullet FPG \qquad \text{Eq 1.}$$

where

$$S(t) = 1 + \frac{S_{max} \bullet C_P^{\gamma}}{SC_{50}^{\gamma} + C_P^{\gamma}} \qquad \text{Eq 2.}$$

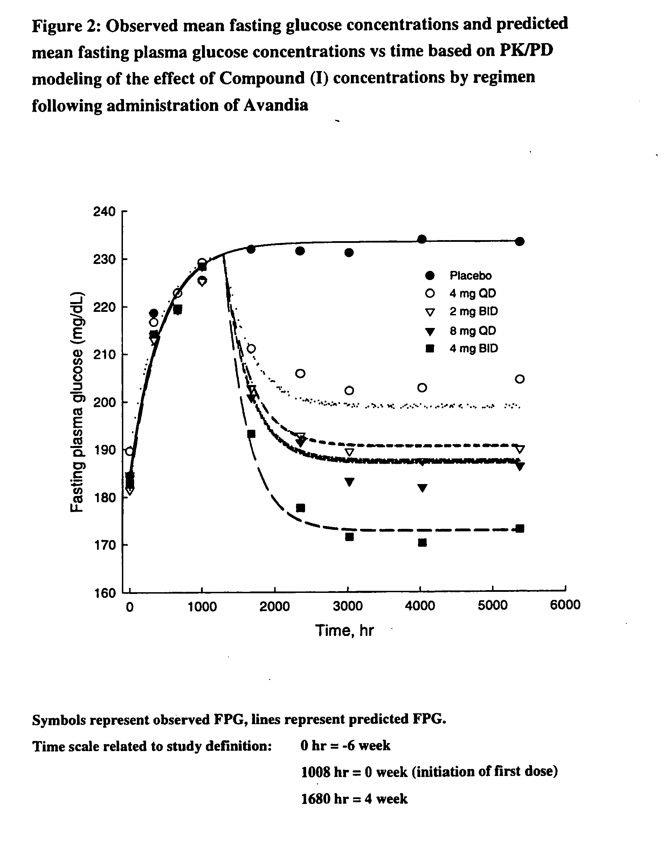

The mean fasting plasma glucose profiles from 6 weeks prior to dosing (time 0) through 26 weeks of treatment for the 5 treatment groups are shown in Figure 2. The PK/PD model accommodates the full nature of the fasting plasma glucose response over the duration of the study period as evidenced by the close agreement between observed and predicted fasting plasma glucose concentrations (Figure 2). An estimated lag-time (292 hours) between the first dose and onset of response was incorporated into the modeling. The lag-time allows the model to describe the slow onset of action of Compound (I) observed over the first 4 weeks of dosing.

The fitted plasma glucose concentrations at steady-state reflect the difference in response to varying total daily dose as well as different dosing frequencies (i.e. once vs twice daily) (Figure 2). The estimated pharmacodynamic parameter values are shown in the table below:

| Parameter | Estimate | CV % |
|---|---|---|
| kin, mg/dL of FPG per h | 0.54 | 4.5 |
| kout, $h^{-1}$ | 0.0023 | 4.5 |
| Smax | 0.44 | 7.1 |
| $SC_{50}$ | 51.4 | 10.7 |
| $\gamma$ | 3.1 | 36.6 |

Based on these data, the $S_{max}$ of the model suggests a maximum FPG reduction of 160 mg/dL.

Although clinically meaningful reductions in glycaemia are evident with once daily dosing of Compound (I), the observation from study 024 that twice daily dosing tended to be more efficacious than once daily can be explained by differences in the concentration-time profiles of Compound (I) across these dosage regimens. Following twice daily dosing of 4 mg, Compound (I) concentrations remain above the $SC_{50}$ for approximately 21 hours compared with only 14 hours following once daily dosing with 8 mg (Figure 1).

Conclusion

The differential effects on FPG reduction following once vs twice daily dosing are well described with a PK/PD model.

References

D'Argenio, DZ and Schumitzky, A (1979). A Program Package for Simulation and Parameter Estimation in Pharmacokinetics. Computer Programs in Biomedicine. 9:115-1134.

Dayneka NL, Garg V and Jusko WJ (1993). Comparison of Four Basic Models of Indirect Pharmacodynamic Responses. Journal of Pharmacokinetics and Biopharmaceutics. Vol 21 (No.4): 457-478.

**Claims**

1. A sustained release pharmaceutical composition in unit dosage form and adapted for oral administration comprising 2 to 12 mg of 5-[4-[2-(N-naethyl-N-(2-pyridyl)amino)ethoxy]benzyl]thiazolidine-2,4-dione or a pharmaceutically acceptable salt or solvate thereof and a pharmaceutically acceptable carrier therefor, which dosage form provides a plasma concentration of 5-[4-[2-(N-methyl-N-(2-pyridyl)amino)ethoxy]benzyl]thiazolidine-2,4-dione of at least 50 ng/mL over a period of up to 24 hours.

2. A sustained release pharmaceutical composition according to claim 1, which composition comprises 4 to 8 mg of 5-[4-[2-(N-metlayl-N-(2-pyridyl)amino)ethoxy]benzyl]thiazolidine-2,4-dione or a pharmaceutically acceptable salt or solvate thereof.

3. A sustained release pharmaceutical composition according to claim 1 or claim 2, which composition provides a plasma concentration of 5-[4-[2-(N-methyl-N-(2-pyridyl)amino)ethoxy]benzyl]thiazolidine-2,4-dione of at least 50 ng/mL over a period of 16 hours.

4. A sustained release pharmaceutical composition according to claim 1 or claim 2, which composition provides a plasma concentration of 5-[4-[2-(N-methyl-N-(2-pyridyl)amino)ethoxy]benzyl]thiazolidine-2,4-dione of at least 50 ng/mL over a period of 12 hours.

5. A sustained release pharmaceutical composition according to any preceding claim, which composition provides a plasma concentration of 5-[4-[2-(N-methyl-N-(2-pyridyl)amino)ethoxy]benzyl]thiazolidine-2,4-dione within the range of 50 to 200 ng/mL.

6. A sustained release pharmaceutical composition according to claim 5, which composition provides a plasma concentration of 5-[4-[2-(N-methyl-N-(2-pyridyl)amino)ethoxy]benzyl]thiazolidine-2,4-dione within the range of 50 to 120 ng/mL.

7. A sustained release pharmaceutical composition according to claim 5, which composition provides a plasma concentration of 5-[4-[2-(N-methyl-N-(2-pyridyl)amino)ethoxy]benzyl]thiazolidine-2,4-dione within the range of 90 to 110 ng/mL.

8. A sustained release pharmaceutical composition according to claim 1, in tablet or capsule form.

9. A sustained release pharmaceutical composition according to any preceding claim, wherein 5-[4-[2-(N-methyl-N-(2-pyridyl)amino)ethoxy]benzyl]thiazolidine-2,4-dione is in the form of a maleate salt.

10. Use of a sustained release pharmaceutical composition according to any preceding claim in the manufacture of a medicament for the treatment of Type 2 diabetes mellitus and conditions associated with diabetes mellitus.

**Patentansprüche**

1. Depotarzneimittel in Einheitsdosierungsform und für die orale Verabreichung adaptiert, umfassend 2 bis 12 mg 5-[4-[2-(N-Methyl-N-(2-pyridyl)amino)ethoxy]-benzyl]thiazolidin-2,4-dion oder ein pharmazeutisch verträgliches Salz oder Solvat davon und einen pharmazeutisch verträglichen Träger dafür, wobei die Dosierungsform eine Plamniakonzeritration des 5-[4-(2-(N-Methyl-N-(2-pyridyl)amino)etboxy]berizyl]-thiazolidin-2,4-dions von mindestens 50 ng/ml über einen Zeitraum von bis zu 24 Stunden bereitstellt.

**2.** Depotarzneimittel gemäß Anspruch 1, wobei die Zusammensetzung 4 bis 8 mg 5-[4-[2-(N-Methyl-N-(2-pyddyl)amino)etrioxy]benzyl]thiazolidin-2,4-dion oder ein pharmazeutisch verträgliches Salz oder Solvat davon umfasst.

**3.** Depotarzneimittel gemäß Anspruch 1 oder Anspruch 2, wobei die Zusammensetzung eine Plasmakonzentration des 5-[4-[2-(N-Methyl-N-(2-pyridyl)amsno)athoxy]-benzyl]thiazolidin-2,4-dions von mindestens 50 ng/ml über einen Zeitraum von 16 Stunden bereitstellt.

**4.** Depotarzneimittel gemäß Anspruch 1 oder Anspruch 2, wobei die Zusammensetzung eine Plasmakonzentration des 5-[4-[2-(N-Methyl-N-(2-pyridyl)amino)etboxy]-benzyl]thiazolidin-2,4-dions von mindestens 50 ng/ml über einen Zeitraum von 12 Stunden bereitstellt.

**5.** Depotarzneimittel gemäß einem der vorstehenden Ansprüche, wobei die Zusammensetzung eine Plasmakonzentration des 5-[4-[2-(N-Methyl-N-(2-pyridyl)amino)ethoxy]benzyl]thiazolidin-2,4-dions im Bereich von 50 bis 200 ng/ml bereitstellt.

**6.** Depotarzneimittel gemäß Anspruch 5, wobei die Zusammensetzung eine Plasmakonzentration des 5-[4-[2-(N-Methyl-N-(2-pyridyl)amino)ethoxy]benzyl]-thiazolidin-2,4-dions im Bereich von 50 bis 120 ng/ml bereitstellt.

**7.** Depotarzneimittel gemäß Anspruch 5, wobei die Zusammensetzung eine Plasmakonzentration des 5-[4-[2-(N-Methyl-N-(2-pyridyl)amino)ethoxy]benzyl]-thiazolidin-2,4-dions im Bereich von 90 bis 110 ng/ml bereitstellt.

**8.** Depotarzneimittel gemäß Anspruch 1 in Tabletten- oder Kapselform.

**9.** Depotarzneimittel gemäß einem der vorstehenden Ansprüche, wobei 5-[4-[2-(N-Mathyl-N-(2-pyridyl)amino)ethoxy]benzyl]thiazolidin-2.4-dion in Form eines Maleatsalzes vorliegt.

**10.** Verwendung eines Depotarzneimittels gemäß einem der vorstehenden Ansprüche zur Herstellung eines Medikaments zur Behandlung von Typ-2-Diabetes mellitus und mit Diabetes mellitus verbundenen Zuständen.

## Revendications

**1.** Composition pharmaceutique à libération prolongée, sous une forme posologique unitaire, et adaptée à l'administration orale, comprenant 2 à 12 mg de 5-[4-[2-(N-méthyl-N-(2-pyridyl)amino)éthoxy]benzyl]thiazolidine-2,4-dione ou d'un de ses sels ou produits de solvatation pharmaceutiquement acceptables et un support pharmaceutiquement acceptable à cette fin, forme posologique qui fournit une concentration plasmatique de 5-[4-[2-(N-méthyl-N-(2-pyridyl)amino)éthoxy]-benzyl]thiazolidine-2,4-dione d'au moins 50 ng/ml en une période de temps allant jusqu'à 24 heures.

**2.** Composition pharmaceutique à libération prolongée suivant la revendication 1, composition qui comprend 4 à 8 mg de 5-[4-[2-(N-méthyl-N-(2-pyridyl)amino)éthoxy]benzyl]thiazolidine-2,4-dione ou d'un de ses sels ou produits de solvatation pharmaceutiquement acceptables.

**3.** Composition pharmaceutique à libération prolongée suivant la revendication 1 ou la revendication 2, composition qui fournit une concentration plasmatique de 5-[4-[2-(N-méthyl-N-(2-pyridyl)amino)éthoxy]benzyl]thiazolidine-2,4-dione d'au moins 50 ng/ml en une période de temps de 16 heures.

**4.** Composition pharmaceutique à libération prolongée suivant la revendication 1 ou la revendication 2, composition qui fournit une concentration plasmatique de 5-[4-[2-(N-méthyl-N-(2-pyridyl)amino)éthoxy]benzyl]thiazolidine-2,4-dione d'au moins 50 ng/ml en une période de temps de 12 heures.

**5.** Composition pharmaceutique à libération prolongée suivant l'une quelconque des revendications précédentes, composition qui fournit une concentration plasmatique de 5-[4-[2-(N-méthyl-N-(2-pyridyl)amino)éthoxy]benzyl]thiazolidine-2,4-dione comprise dans l'intervalle de 50 à 200 ng/ml.

**6.** Composition pharmaceutique à libération prolongée suivant la revendication 5, composition qui fournit une concentration plasmatique de 5-[4-[2-(N-méthyl-N-(2-pyridyl)-amino)éthoxy]benzyl]thiazolidine-2,4-dione comprise dans l'intervalle de 50 à 120 ng/ml.

**7.** Composition pharmaceutique à libération prolongée suivant la revendication 5, composition qui fournit une concentration plasmatique de 5-[4-[2-(N-méthyl-N-(2-pyridyl)-amino)éthoxy]benzyl]thiazolidine-2,4-dione comprise dans l'intervalle de 90 à 110 ng/ml.

**8.** Composition pharmaceutique à libération prolongée suivant la revendication 1, sous forme de comprimé ou de capsule.

**9.** Composition pharmaceutique à libération prolongée suivant l'une quelconque des revendications précédentes, dans laquelle la 5-[4-[2-(N-méthyl-N-(2-pyridyl)amino)éthoxy]-benzyl]thiazolidine-2,4-dione est sous forme d'un sel maléate.

**10.** Utilisation d'une composition pharmaceutique à libération prolongée suivant l'une quelconque des revendications précédentes dans la production d'un médicament destiné au traitement du diabète sucré de type 2 et des affections associées au diabète sucré.

**Figure 1: Simulated steady-state concentrations of Compound (I) (upper) and M10 (lower) over a 24 hour dosing interval following 4 mg and 8 mg total daily doses of Avandia**

Figure 2: Observed mean fasting glucose concentrations and predicted mean fasting plasma glucose concentrations vs time based on PK/PD modeling of the effect of Compound (I) concentrations by regimen following administration of Avandia

Symbols represent observed FPG, lines represent predicted FPG.

Time scale related to study definition: 0 hr = -6 week

1008 hr = 0 week (initiation of first dose)

1680 hr = 4 week

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0306228 A
- WO 9405659 A
- EP 0008203 A
- EP 0139421 A
- EP 0032128 A
- EP 0428312 A
- EP 0489663 A
- EP 0155845 A
- EP 0257781 A
- EP 0208420 A
- EP 0177353 A
- EP 0319189 A
- EP 0332331 A
- EP 0332332 A
- EP 0528734 A
- EP 0508740 A
- WO 9218501 A
- WO 9302079 A
- WO 9322445 A
- US 5104888 A
- US 5478852 A

**Non-patent literature cited in the description**

- **TUESCHER A ; RICHTERICH, P.** *Schweiz. med. Wschr,* 1971, vol. 101, 345, 390
- **FRANK P.** Monitoring the Diabetic Patent with Glycosolated Hemoglobin Measurements. *Clinical Products,* 1988
- **DAYNEKA NL ; GARG V ; JUSKO WJ.** Comparison of Four Basic Models of Indirect Pharmacodynamic Responses. *J of Pharmacokinetics and Biopharmaceutics,* 1993, vol. 21 (4
- **D'ARGENIO, DZ ; SCHUMITZKY, A.** A Program Package for Simulation and Parameter Estimation in Pharmacokinetics. *Computer Programs in Biomedicine,* 1979, vol. 9, 115-1134
- **DAYNEKA NL ; GARG V ; JUSKO WJ.** Comparison of Four Basic Models of Indirect Pharmacodynamic Responses. *Journal of Pharmacokinetics and Biopharmaceutics,* 1993, vol. 21 (4), 457-478